(19) Europäisches Patentamt
European Patent Office
Office européen des brevets

(11) Veröffentlichungsnummer: **0 483 106 B1**

# (12) EUROPÄISCHE PATENTSCHRIFT

(45) Veröffentlichungstag der Patentschrift: **31.08.94**

(51) Int. Cl.$^{5}$: **C07C 63/72**, C07C 51/265

(21) Anmeldenummer: **92100514.6**

(22) Anmeldetag: **17.11.88**

(60) Veröffentlichungsnummer der früheren Anmeldung nach Art. 76 EPÜ: **0 317 884**

## (54) Verfahren zur Herstellung teilfluorierter Tetracarbonsäuren.

(30) Priorität: **24.11.87 DE 3739796**
**24.11.87 DE 3739800**

(43) Veröffentlichungstag der Anmeldung:
**29.04.92 Patentblatt 92/18**

(45) Bekanntmachung des Hinweises auf die Patenterteilung:
**31.08.94 Patentblatt 94/35**

(84) Benannte Vertragsstaaten:
**BE CH DE FR GB IT LI NL**

(56) Entgegenhaltungen:
**EP-A- 0 261 892**
**DE-A- 1 418 295**

(73) Patentinhaber: **HOECHST AKTIENGESELLSCHAFT**

**D-65926 Frankfurt (DE)**

(72) Erfinder: **Röhrscheid, Freimund, Dr.**
**Amselweg 24**
**W-6233 Kelkheim (Taunus) (DE)**
Erfinder: **Siegemund, Günter, Dr.**
**Frankfurter Strasse 21**
**W-6238 Hofheim am Taunus (DE)**
Erfinder: **Lau, Jürgen, Dr.**
**Breitenwiesenstrasse 24**
**W-8751 Haibach (DE)**

EP 0 483 106 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren zur Herstellung von teilfluorierten Tetracarbonsäuren und ihren Dianhydriden mit verschiedenen Fluorgehalten.

Die Herstellung von 1,1-Bis-[4-(1,2-dicarboxyphenyl)]-1-phenyl-2,2,2-trifluorethan aus der entsprechenden 1,2-Dimethylphenylverbindung ist bekannt (CA 107: 97277 j (1987), NASA, Techn. Memory 87 113 (1985)). Die Oxidation wurde mit Kaliumpermanganat durchgeführt und ergab niedrige Ausbeuten und ein verunreinigtes sehr hygroskopisches Produkt (3F-Tetracarbonsäure).

Dixylylhexafluorpropan (DX-F6) und seine Oxidation mit Kaliumpermanganat in einer Mischung aus Pyridin und Wasser zum Kaliumsalz des 2,2-Bis-(3,4-dicarboxyphenyl)hexafluorpropans (6F-Tetracarbonsäure) wird in der US-A 3.310.573 beschrieben. Die Art der Oxidation benötigt einen hohen Chemikalienbedarf, die Isolierung der Tetracarbonsäure ist auch hier sehr umständlich, und das verwendete Lösungsmittelsystem sowie das Manganoxid müssen aufgearbeitet werden.

Die Oxidation von 2,2-Bis(4-methylphenyl)hexafluorpropan mit Luftsauerstoff in essigsaurem Medium in Gegenwart eines Katalysators aus Kobalt- und Bromionen ist bekannt (SU-644 777 = CA 90:P 168310 K (1979). Die Übertragung dieser Reaktion auf fluorhaltige Verbindungen, die zwei Hexafluorisopropylidenbrücken und zusätzlich eine Diphenyletherbrücke tragen, lag jedoch nicht nahe, da die Etherbrücke im allgemeinen das Reaktionsverhalten bei Oxidationsreaktionen verändert. Auch die Übertragung dieser Reaktion auf fluorhaltige Verbindungen, die eine 3,4-Dimethylphenylkonfiguration aufweisen, scheiterte aber, da die Ausbeute und Reinheit der erhaltenen Verbindungen nicht befriedigend war.

Ferner ist ein Verfahren zur Herstellung von Benzoltetracarbonsäuren und ihren Anhydriden durch Oxidation von tetraalkyl-substituierten Benzolen mit Sauerstoff oder freien Sauerstoff enthaltenden Gasen in Gegenwart von Schwermetallverbindungen und Bromverbindungen bei erhöhten Temperaturen bekannt (DE-A 21 12 009). Aus der Veröffentlichtung geht hervor, daß die vollständige Oxidation von zwei nachbarständigen Methylgruppen erhebliche Probleme bereitet.

Nach Beendigung der Oxidationsreaktion finden sich im Reaktionsprodukt nicht oxidierte Methylgruppen, CHO- und $CH_2OH$-Gruppen. Die $CH_2OH$-Gruppe bildet mit der benachbarten Carboxylgruppe Phthalidringe.

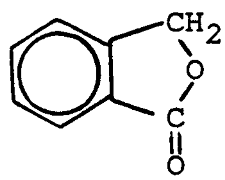

Zusätzlich erschwerend tritt hinzu, daß benachbarte Carboxygruppen mit Schwermetallen stabile Verbindungen bilden und als Metallsalze ausfallen können. Dieses setzt die katalytische Aktivität des Katalysators stark herab. Diese Metallsalzbildung wird sowohl bei der Phthalsäure als auch bei der Pyromellithsäure beobachtet.

Zur Verhinderung des Absinkens der katalytischen Aktivität des Katalysators und zur Unterstützung der Oxidation insbesondere einer vierten Alkylgruppe sei es erforderlich, daß bei der Reaktion große Mengen an Bromionen anwesend sein müssen.

Eine Übertragung dieses Verfahrens auf fluorhaltige Verbindungen ist aber nicht möglich, da durch Bromierungsreaktionen unter den angegebenen Versuchsbedingungen Produkte mit hohem Bromgehalt erhalten werden, die schwer abtrennbar sind und das Endprodukt nicht in genügender Reinheit und Ausbeute erhalten lassen. Darüber hinaus ist festgestellt worden, daß die teilfluorierten Polycarbonsäuren aus der Reaktionslösung gar nicht oder nur unvollständig auskristallisieren und zudem teilweise mit ihren Schwermetallsalzen vermischt sind, die eine Reinigung erheblich erschweren.

Dieses Verhalten ist um so überraschender, weil Polycarbonsäuren wie Terephthalsäure, Phthalsäure oder Pyromellithsäure sofort und in gut kristallisierter Form aus dem Reaktionsmedium wie Essigsäure auskristallisieren.

Es bestand daher die Aufgabe, ein Verfahren für teilfluorierte Carbonsäuren zu schaffen, das hohe Ausbeuten und eine hohe Reinheit der erhaltenen Produkte ermöglicht. So sind die teilfluorierten Tetracarbonsäuren und ihre Dianhydride beispielsweise Bausteine für Polyimide, die für technisch anspruchsvolle Zwecke verwendet werden, z.B. für thermisch hochbelastbare Überzüge und Klebstoffe im Flugzeugbau

oder in der Mikroelektronik. Für viele dieser Anwendungsgebiete ist es daher wünschenswert, eine hohe Reinheit der eingesetzten Substanzen, beispielsweise von 99 % oder mehr zu fordern. Die Verwendung der nach dem erfindungsgemäßen Verfahren hergestellten Verbindungen in der Mikroelektronik macht es außerdem erforderlich, daß alle Metallionen, die aus dem Katalysator stammen oder während der Reaktionsschritte eingeschleppt werden, bis auf Konzentrationen im ppm-Bereich entfernt werden.

Die hochselektive Oxidation soll in der möglichst vollständigen Oxidation aller Methylgruppen der Methylverbindungen und in der Verhinderung von Nebenreaktionen wie Decarboxylierung und Kondensation bestehen. Um dieses Ziel zu erreichen, müssen spezielle Oxidationsbedingungen gefunden werden.

Gegenstand der Erfindung ist ein Verfahren zur Herstellung

a) einer Verbindung der Formel

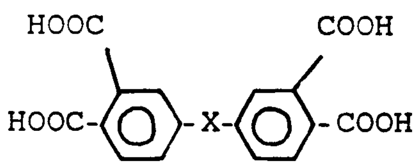

(I)

in der X die Gruppen

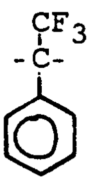

oder

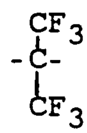

bedeuten, oder deren Anhydrid durch Luftoxidation in saurem Medium bei erhöhtem Druck und erhöhter Temperatur in Gegenwart eines Katalysatorgemischs, dadurch gekennzeichnet, daß die entsprechende Tetramethylverbindung in einem sauren organischen Medium durch Einleiten von Luftsauerstoff bei Temperaturen von 120 bis 220 °C und einem Druck zwischen 5 und 40 bar in Gegenwart von mindestens zwei Schwermetallverbindungen sowie von Bromidionen oxidiert und als solche isoliert wird oder das erhaltene Reaktionsgut in das Dianhydrid der Verbindung der Formel (I) übergeführt wird.

Die Aufgabe der Erfindung wurde gelöst durch Einhalten besonderer Bedingungen für die Oxidation von den Methylverbindungen. Bei den entsprechenden Tetracarbonsäuren wurde eine Umsatzrate von über 90 % erreicht.

Folgende Maßnahmen haben sich dabei als günstig erwiesen:

1) Katalysator:
   - a) Wahl der Bestandteile für den Katalysator, insbesondere $Co^{2+}$, $Mn^{2+}$, $Ce^{3+}$, $Br^{-}$
   - b) optimales Verhältnis der Metallionen zueinander
   - c) hohe Gesamtmetallkonzentration in der Reaktionlösung
   - d) hohes Konzentrationsverhältnis von Metallionen : Bromidionen

2) Reaktionsbedingungen:
   - a) hoher Sauerstoffpartialdruck
   - b) kontrollierter Wassergehalt

Die Oxidation findet in einem sauren organischen Medium statt, bei der die Methylphenylverbindungen mit molekularem Sauerstoff oxidiert werden, wobei das saure Medium zu mindestens 40 % aus einer Monocarbonsäure wie Essigsäure oder Propionsäure oder deren Mischungen besteht. Essigsäure ist wegen ihrer größeren Beständigkeit gegen einen oxidativen Abbau vorzuziehen. Das Verhältnis von saurem Medium zu eingesetzter Ausgangssubstanz kann bis zu einem Verhältnis von 40 : 60 Gew.-%, bezogen auf die Gesamtreaktionsmasse, angewandt werden.

Das Katalysatorgemisch besteht aus mindestens zwei Schwermetallsalzen sowie Bromidionen. Als Schwermetalle werden beispielsweise Kobalt, Mangan oder Cer eingesetzt, wobei die Anwesenheit von Kobalt stets erforderlich ist. Durch die Mischung der Metallsalze kann die Gesamtmetallkonzentration geringer gehalten werden, als wenn nur Kobalt allein eingesetzt wird.

Bromidionen sind für den vollständigen Ablauf der Oxidation unbedingt notwendig. Wird als Metallkomponente ein Gemisch von Kobalt- und Manganionen verwendet, so werden die Metalle im allgemeinen im Mol-Verhältnis von 3 : 1 bis 1 : 3, vorzugsweise 1 : 1 eingesetzt. Die Summe der Konzentrationen der

beiden Elemente beträgt im allgemeinen 0,01 bis 0,2, vorzugsweise 0,02 bis 0,12 und insbesondere 0,04 bis 0,08 Grammatom/kg Gesamtmasse. Das Mol-Verhältnis der Summe von Kobalt und Mangan zu Brom ist im allgemeinen 1 : (0,01 bis 0,8), vorzugsweise 1 : (0,05 bis 0,4). Wie bereits erwähnt, ist es möglich, zusätzlich zu den beiden Metallionen des Katalysators auch Cerionen einzusetzen. Diese katalysieren die Oxidation unvollständig oxidierter Zwischenstufen. Ihre Anwesenheit erhöht die Reinheit und die Ausbeute der teilfluorierten Carbonsäuren. Die Cerionen werden dem Katalysator im Mol-Verhältnis der Summe der Kobalt- und Manganionen zu Cerionen wie 1 : (0,02 bis 1,2), vorzugsweise 1 : (0,05 bis 0,6) zugefügt. Wird ein Gemisch der Metallionen von Kobalt und Cer verwendet, so beträgt das Molverhältnis der beiden Metalle im allgemeinen 1 : (0,02 bis 1,2), wobei sich das Verhältnis der Metalle zu Brom wie vorstehend beschrieben ausweist. Die Molverhältnisse beziehen sich stets auf die Gesamtmasse, d.h. aus der Summe der zu oxidierenden Verbindung, Lösungsmittel und Katalysator. Vorzugsweise werden die Metallionen in Form ihrer Acetate eingesetzt.

Brom kann in Form von Bromiden, z.B. der Bromide der Alkalimetalle, einschließlich dem Ammoniumbromid, und denen der Metalle Kobalt, Mangan und Cer oder als Lösung von Bromwasserstoff in Wasser oder Eisessig eingesetzt werden. Daneben können auch bromhaltige organische Verbindungen verwendet werden, die während der Oxidation zerfallen und Bromionen freisetzen, z.B. Tetrabrommethan. Man kann die Bromidionen-Konzentration in der Reaktionslösung sehr stark - bis zu einem Wert von ca. 20 für das Molverhältnis $\Sigma M^n$:Br (Summe der Metallionen zu Bromionen) - senken, ohne daß es zu einer merklichen Abnahme der Reaktionsgeschwindigkeit kommt. Durch diese Maßnahme, die gleichzeitig auch das Verhältnis der Tetramethylverbindungen : Br in der Reaktionslösung erhöht, wird die unerwünschte Kernbromierung stark verringert.

Die Oxidation wird im allgemeinen bei Temperaturen von 120 bis 220, vorzugsweise 140 bis 190 und insbesondere 155 bis 180 °C durchgeführt. Der Druck im Reaktor liegt im allgemeinen zwischen 5 und 40, vorzugsweise zwischen 10 und 30 und insbesondere zwischen 14 bis 20 bar.

Für die Verfahrensweise ist es günstig, daß die zur Oxidation benötigte Luft nahe dem Boden des Reaktors in die Flüssigphase eingeleitet und durch starkes Rühren oder durch spezielle Düsen in der Flüssigphase fein verteilt wird. Besonders günstig ist es, ein Oxidationsgemisch einzufahren, dessen Sauerstoffgehalt durch Beimischen von reinem Sauerstoff auf einen Anteil von über 21 Volumen-% erhöht wurde. Durch diese Maßnahme werden hohe Sauerstoff-Partialdrucke in den in die Flüssigphase eintretenden Gasblasen erreicht. Es ist vorteilhaft, wenn der Sauerstoff-Partialdruck an der Austrittsstelle der Einleitungsvorrichtung mindestens 1 bar beträgt, vorzugsweise bei 2 bis 15 und insbesondere bei 3 bis 10 bar liegt.

Es ist für die Verfahrensführung weiterhin günstig, daß der Restsauerstoffgehalt des Abgases bestimmte Werte nicht unterschreitet. Der Sauerstoff-Partialdruck wird definiert durch die Formel

$$P_{O_2} = \text{Volumen-\%}\ O_2 \cdot (P_{ges} - P_{ac\text{-}Dampfdruck})$$

d.h. er ist das mathematische Produkt aus dem Restsauerstoffgehalt und der Differenz aus dem Gesamtdruck und dem Essigsäuredampfdruck (ac-Dampfdruck) bei der vorliegenden Reaktionstemperatur. Dieser Sauerstoff-Partialdruck in der Gasphase über der Reaktionslösung soll 0,2 bar nicht unterschreiten und liegt bevorzugt zwischen 0,35 und 2,8, insbesondere zwischen 0,45 und 1,3 bar.

Nach Beendigung der stark exothermen Reaktion ist es zweckmäßig, zur Vervollständigung der Oxidation aller Methylgruppen den Reaktor für 1 bis 3 Stunden, vorzugsweise für 2 Stunden, auf 150 bis 190 °C, vorzugsweise 160 bis 180 °C, bei einem Sauerstoffpartialdruck von 0,4 bis 2 bar, vorzugsweise 0,5 bis 1,3 bar zu halten.

Einen wesentlichen Einfluß auf die Durchführung des erfindungsgemäßen Verfahrens hat die Wasserkonzentration des sauren Mediums, in dem die Reaktion durchgeführt wird. Zwar können die Tetramethylverbindungen auch in - beispielsweise - Essigsäure mit einer Wasserkonzentration von 15 % und mehr oxidiert werden, jedoch vermindert sich dabei Ausbeute und vor allem die Reinheit der erhaltenen Produkte, und die Oxidation aller vier Methylgruppen läuft nur noch unvollständig ab. Andererseits wurde festgestellt, daß in wasserfreier Essigsäure die Metallionen des Katalysators durch die Tetracarbonsäuren ausgefällt und damit inaktiviert werden. Der Bereich für die Wasserkonzentration, in dem die Metallionen gelöst bleiben und in dem die Oxidation genügend vollständig abläuft, liegt zwischen 2 und 12, vorzugsweise zwischen 2 und 7 und insbesondere zwischen 3 und 5 % Wasser in der Monocarbonsäure z.B. Essigsäure.

Es wurde gefunden, daß überraschenderweise die Dianhydride der Tetracarbonsäuren in Eisessig oder Mischungen aus Eisessig und Acetanhydrid wenig löslich sind, wenn man durch geeignete Methoden das

Wasser aus der Reaktionslösung entfernt und die Tetracarbonsäuren in ihre Anhydride überführt. Diese Umwandlung kann durch Destillation und/oder durch Zugabe von Essigsäureanhydrid geschehen. Die Dianhydride kristallisieren in hoher Ausbeute und gut filtrierbarer Form aus und wurden durch Waschen mit Eisessig, vorzugsweise einer Mischung aus Eisessig und Acetanhydrid, von den Metallsalzen und den löslichen Nebenprodukten befreit. Das Waschen mit einer Mischung aus Eisessig und Acetanhydrid ist besonders geeignet, da diese Mischung das Verbacken des Filterkuchens verhindert. Die Dianhydride fallen mit einer Reinheit von 94 bis 97 % an.

Überraschenderweise lösen sich auch die Metallsalze während der Anhydridbildung auf, so daß schon nach dieser ersten Reinigungsoperation der Gehalt aller Metallionen bei 50 bis 100 ppm liegt.

Die Umwandlung der Tetracarbonsäuren führt man vorzugsweise so aus, daß man aus der Reaktionslösung ein Gemisch von Essigsäure und Wasser abdestilliert und anschließend in der Hitze Acetanhydrid in einem kleinen Überschuß über die berechnete Menge Acetanhydrid zufügt (ca. 3 bis 12 % Acetanhydrid in der Lösung).

Eine besondere Verfahrensvariante besteht darin, daß bei erhöhter Temperatur und unter Druck von der Reaktionslösung über eine Kolonne Wasser abdestilliert wird. Unter diesen Bedingungen bilden sich ebenfalls die Dianhydride der Tetracarbonsäuren unter Wasserabspaltung. Gleichzeitig werden auch die Metallsalze wieder gelöst. Zur Vervollständigung der Umsetzung zum Dianhydrid wird am Ende soviel Acetanhydrid zugefügt, daß sich ca. 3 bis 12 % Acetanhydrid in der Lösung befinden.

Die Bildung von Dianhydrid durch destillative Entfernung von Wasser wird vorzugsweise bei einer Temperatur oberhalb 140 °C, nötigenfalls unter dem zusätzlichen Druck eines Inertgases durchgeführt. Die Essigsäure kann auch ganz oder teilweise durch eine andere aliphatische Carbonsäure wie Propionsäure, Hexansäure oder 2-Ethylhexansäure ersetzt werden.

Im allgemeinen werden die filtrierten und gewaschenen Dianhydride im Luftstrom, vorzugsweise unter vermindertem Druck, bei erhöhter Temperatur getrocknet.

Bei der Isolierung als Tetracarbonsäure ist es zweckmäßig, in Gegenwart einer anorganischen oder organischen Säure z.B. Salzsäure, zu arbeiten. Die Tetracarbonsäuren können vorteilhaft in einer gut filtrierbaren Form aus Wasser auskristallisiert werden, wenn geringe Konzentrationen von Essigsäure darin gelöst sind, vorzugsweise 6 bis 12 Gew.-% Essigsäure. Im allgemeinen wird so dabei verfahren, daß nach beendeter Oxidation aus der Reaktionslösung soviel Essigsäure abdestilliert wird, bis die Sumpftemperatur ca. 130 bis 155 °C beträgt und die Schmelze noch gut rührbar ist. In die heiße Schmelze wird heißes Wasser und gegebenenfalls Säure zugesetzt und die Lösung noch einmal, vorzugsweise unter Druck bis zu 2 Stunden auf 130 bis 150 °C erhitzt.

Die Tetracarbonsäuren kristallisieren als Hydrat aus und können durch vorsichtiges Trocknen bei Raumtemperatur als Hexahydrat isoliert werden. Durch Erhitzen auf 50 bis 80 °C im Gasstrom werden sie in die Tetracarbonsäuren und durch Erhitzen auf 180 bis 190 °C unter vermindertem Druck in die Dianhydride umgewandelt.

Besonders reine Produkte werden erhalten, wenn die noch wasserfeuchte Tetracarbonsäure in einem Lösungsmittel suspendiert und das Wasser abdestilliert wird. Dabei bildet sich zuerst die hydratfreie Tetracarbonsäure, aus der bei weiterer Temperaturerhöhung das Dianhydrid entsteht.

Besonders geeignet sind aromatische Lösemittel wie Toluol, o-Xylol, Tetrahydronaphthalin, Acetophenon oder Diphenylether.

Die Anhydridbildung in aromatischen Lösemitteln kann durch Zugabe katalytischer Mengen an Carbonsäuren, z.B. aliphatischen Carbonsäuren wie Essigsäure, 2-Ethylhexansäure, oder anderer Säuren wie Toluolsulfonsäure erheblich beschleunigt werden.

Die Tetracarbonsäuren können zur Herstellung von Polykondensaten wie Polyimiden, Polycarbonsäureamiden, Polyamidcarbonsäureestern, Polyamiden und Imidoligomeren eingesetzt werden, die u.a. niedrige Schmelzpunkte, hohe Löslichkeit, niedrige Dielektrizitätskonstanten, erhöhte thermische Stabilität aufweisen.

In den nachfolgenden Beispielen sind % stets Gewichtsprozent.

## Beispiele

### 1) **1,1-Bis-(3,4-dicarboxyphenyl)-1-phenyl-2,2,2-trifluorethan (3F-Tetracarbonsäure)**

Eine Mischung aus 148,3 g 1,1-Bis-(3,4-dimethylphenyl)-1-phenyl-2,2,2-trifluorethan, 2,49 g $Co(OAc)_2 \cdot 4 H_2O$, 2,45 g $Mn(OAc)_2 \cdot 4 H_2O$, 0,41 g HBr entsprechend 4,1 g einer 10 %igen HBr-Lösung in Eisessig und 550 g Eisessig wurde unter 7,5 bar Sauerstoffdruck auf 180 bis 185 °C erhitzt. Ab ca. 100 °C setzte die exotherme Reaktion ein, die ca. 85 Minuten anhielt. Anschließend wurde die Temperatur noch für 1 Stunde auf 176 °C gehalten. Es resultierten 784 g Lösung. Über einen absteigenden Kühler wurden an der

Reaktionslösung unter Rühren ca. 530 g Essigsäure-Wasser abdestilliert, wobei die Sumpftemperatur auf 145 °C stieg. Zur heißen Schmelze wurden 150 g Wasser von 80 °C hinzugegeben, die Mischung mit 50 ml konzentrierter Salzsäure versetzt und für eine Stunde zum Sieden erhitzt. Unter starkem Rühren wurde abgekühlt und der Ansatz mit Kristallen der 3F-Tetracarbonsäure angeimpft. Die Kristallsuspension wurde abgesaugt und viermal mit 50 ml 2 n Salzsäure und zweimal mit 25 ml Wasser gewaschen. Die feuchten Kristalle wurden bei 50 °C/64 mbar im Luftstrom getrocknet.

Ausbeute: 190,4 g (96,8 % d. Th.) 3F-Tetracarbonsäure Fp. 210 bis 213 °C, Wasserabspaltung; Carboxylgruppengehalt: 8,08 mVal COOH/g (ber. 8,20)

2) **1,1-Bis-(3,4-dicarboxylphenyl)-1-phenyl-2,2,2-trifluorethan-dianhydrid (3F-Dianhydrid)**

Aus einer Reaktionslösung eines gleichen Oxidationsansatzes wie in Beispiel 1, wurden unter Rühren über einen absteigenden Kühler 400 g Essigsäure-Wasser-Gemisch abdestilliert. Anschließend wurde bei Siedetemperatur eine Mischung aus 122,4 g Acetanhyrid und 120 g Eisessig im Laufe einer halben Stunde zugetropft und eine Stunde am Rückfluß gekocht. Beim Abkühlen unter Rühren setzte die Kristallisation unterhalb 85 °C ein. Nach 4 Stunden wurde abgesaugt und dreimal mit je 25 ml einer Mischung aus 95 % Eisessig und 5 % Essigsäureanhydrid gewaschen. Die Kristalle wurden bei 80 °C/65 mbar im leichten Luftstrom getrocknet.

Ausbeute: 119,0 g (65,3 % d.Th.) 3F-Dianhydrid, gelbliche Kristalle; Festpunkt: 204 bis 205,5 °C
Aus den vereinigten Filtraten wurden weitere 36,6 g (20,1 % d. Th.) 3F-Dianhydrid mit einem Fp. von 201 bis 204 °C gewonnen.

**3) 2,2-Bis-(3,4-dicarboxyphenyl)-hexafluorpropan (6F-Tetracarbonsäure)**

In einem 1 l-Glasautoklaven, der mit Dosierpumpe, Thermometer, Rührer und Rückflußkühler ausgestattet war, wurde eine Lösung von 2,5 g Kobaltacetattetrahydrat, 2,45 g Manganacetattetrahydrat und 0,44 g Bromwasserstoff in 311 g Eisessig vorgelegt. Parallel dazu wurde eine Lösung von 180,2 g Dixylylhexafluorpropan in einem Gemisch von 102 g Acetanhydrid und 60 g Eisessig in einer Dosiereinrichtung bereitgestellt. Der Autoklav wurde durch Einleiten von Sauerstoff unter einen Gesamtdruck von 7,5 bar gesetzt, ein Abgaswert von 30 Nl/h (Nl = Normliter) eingestellt und der Inhalt erhitzt. Bei ca. 160 °C wurde mit der Zudosierung von Dixylylhexafluorpropan begonnen und innerhalb von 100 Minuten die Gesamtmenge hinzugefügt. Die Temperatur der exothermen Reaktion wurde bei 170 bis 175 °C gehalten und der Ansatz nach Beendigung der Dosierung für eine weitere Stunde bei 175 °C durch Heizen gehalten. Von der Reaktionsmischung (ca. 790 bis 800 g) wurden bei Normaldruck ca. 500 g Essigsäure-Wasser-Gemisch abdestilliert. Sobald die Temperatur des Rückstandes auf 145 °C gestiegen war, wurde der Autoklav durch Einleiten von Stickstoff auf einen Druck von 4 bar gesetzt, 500 g destilliertes Wasser zugegeben und die Mischung für eine Stunde bei 145 °C gehalten. Anschließend wurde bis auf Raumtemperatur abgekühlt und die gebildeten Kristallblättchen abgesaugt. Die Mutterlauge enthielt 6 bis 10 % Essigsäure. Der Filterkuchen wurde 8mal mit je 50 ml Eiswasser gewaschen. Das feuchte Produkt, das aus 6F-Tetracarbonsäurehexahydrat und anhaftendem Wasser bestand, wurde für 8 Stunden bei 40 bis 50 °C/50 mbar im Luftstrom getrocknet. Anschließend wurde die Temperatur auf 80 °C erhöht und der Feststoff für weitere 12 Stunden getrocknet. Ausbeute 212,3 g (88,4 % d.Th.) 6F-Tetracarbonsäure, Fp.: 231 bis 234 °C (Wasserabspaltung), Carboxylgruppengehalt: 8,45 mVal COOH g (ber. 8,33), Schüttgewicht 0,30 g/$cm^2$, Restionengehalt (Mikrogramm/g): Kobalt 1, Mangan 1, Brom 189.

**4) 2,2-Bis-(3,4-dicarboxyphenyl)-hexafluorpropan-dianhydrid**

Gemäß der Verfahrensweise im Beispiel 3 wurden 2,24 g Kobaltacetattetrahydrat, 2,21 g Manganacetattetrahydrat und 0,365 g Bromwasserstoff in 302 g Eisessig mit einer Lösung von 142,6 g Dixylylhexafluorpropan in 125 g Eisessig zur Reaktion gebracht. Der Sauerstoffdruck betrug 8 bar und die Reaktionstemperatur während der Oxidation wurde im Bereich von 165 bis 175 °C gehalten. Aus der erhaltenen Reaktionslösung wurde unter Rühren solange Essigsäure und Wasser abdestilliert, bis eine Sumpftemperatur von 130 °C errreicht war. Dann wurden bei dieser Temperatur innerhalb von 20 Minuten eine Mischung aus 92 g Acetanhydrid und 200 g Eisessig hinzugefügt. Die ca. 5 Gew.-% Acetanhydrid enthaltende Flüssigkeit wurde eine Stunde am Rückfluß gekocht, auf 20 °C abgekühlt und bei dieser Temperatur für 2 1/2 Stunden gerührt. Nach dem Absaugen wurde der Filterkuchen achtmal mit 50 g einer Mischung aus Eisessig und Acetanhydrid (95 : 5) gewaschen und die erhaltenen Kristalle bei 100 °C und 60 mbar im schwachen Luftstrom getrocknet. Ausbeute 165,5 g (94,7 %) farblose Kristalle, Sinterbeginn 237 °C, Fp.: 242 bis

243 °C, Reinheit 95,8 % 6F-Dianhydrid, Verunreinigung durch Katalysatorspuren: 10 ppm Kobalt, 6 ppm Mangan, 215 ppm Brom.

**5) 6F-Dianhydrid aus 6F-Tetracarbonsäure**

212,3 g 6F-Tetracarbonsäure wurden im Kolben eines Rotationsverdampfers in einem Ölbad bei 190 °C bewegt. Der gebildete Wasserdampf wurde durch einen leichten Luftstrom aus dem Kolben verdrängt. In einer Kühlfalle wurden 15,1 g Wasser aufgefangen. Ausbeute 198,5 g 6F-Dianhydrid, Fp. 242 bis 243,5 °C, Anhydridgruppen 4,51 mbar (berechnet 4,50).

**6) 6F-Dianhydrid aus wasserfeuchter 6F-Tetracarbonsäure**

342,2 g wasserfeuchte 6F-Tetracarbonsäure, erhalten gemäß Beispiel 3, wurden in einem Kolben, der mit Rührer und Wasserabscheider ausgerüstet war, in 700 g Tetrahydronaphthalin suspendiert. Der Ansatz wurde unter starkem Rühren erhitzt und ca. 125 g Wasser abdestilliert. Dann wurde die Temperatur bis zum Siedepunkt gesteigert und langsam Tetrahydronaphthalin, das noch restliches abgespaltenes Wasser mitführt, abdestilliert. Die Anhydridbildung war beendet, wenn kein Wasser mehr abgeschieden wurde. Die ausgeschiedenen Kristalle wurden bei 20 °C mit Tetrahydronaphthalin gewaschen und bei 100 °C unter vermindertem Druck getrocknet. Ausbeute 206,4 g (85,9 % d.Th.), Reinheit 99,9 %, Fp.: 243 bis 245 °C.

In den nachfolgenden Beispielen 7 bis 31, die unter den Bedingungen und der Verfahrensweise des Beispiels 4 durchgeführt wurden, wird die Überlegenheit des erfindungsgemäßen Verfahrens eindeutig demonstriert. So wird in den Beispielen 7 bis 13 (Tabelle 1) der Einfluß der Katalysatorzusammensetzung deutlich. Unter den erfindungsgemäßen Bedingungen werden Produkte mit hoher Reinheit in guter Ausbeute erhalten.

In den Beispielen 14 bis 20 (Tabelle 2) wird der Einfluß der Ionenkonzentration und des Verhältnisses von Kobalt zu Mangan demonstriert. Es ist ersichtlich, daß bei niedriger Gesamtmetallionenkonzentration eine Anhebung der Bromionenkonzentration zwar eine Erhöhung der Ausbeute bewirkt, diese jedoch nicht auf den Stand gebracht werden kann, wie er durch die Maßnahmen der Erfindung erhalten wird. Auch die Beispiele 21 bis 25 (Tabelle 3) spiegeln noch einmal den Einfluß der Bromidionenkonzentration wieder. Je höher diese ist, desto größer ist der Anteil an organisch gebundenem Brom in den Endprodukten. Ist das Verhältnis der Summe der Metallionen zu den Bromionen sehr groß, so sinkt die Ausbeute erheblich. In den Beispielen 26 bis 31 (Tabelle 4) wird deutlich, daß die Wasserkonzentration bereits bei 15 % Wassergehalt einen deutlichen Abfall der Ausbeute bewirkt.

Die für die Umsetzung benötigten Substanzmengen ergeben sich aus der Gesamtmasse des Ansatzes und den in der Tabelle angegebenen Konzentrationen. Diese Konzentrationen der Stoffe sind in [Mol/kg] auf die Gesamtmasse aller eingesetzten Reaktionsbestandteile (außer $O_2$) bezogen. Die Essigsäure ergibt sich dabei als Restmenge zur Gesamtmasse, z.B. entspricht dem Wert $C_{DX-F6}$ = 0,66 Mol/kg in der Tabelle bei 0,6 kg Gesamtmasse die Dixylylhexafluorpropan (DX-F6)-Menge von 0,396 Mol oder 142,7 g. Wie im Beispiel 4 wird das DX-F6 während der Reaktion eindosiert. Für die Bereitung dieser Lösung wird 1/3 der Essigsäuregesamtmenge verwendet.

**Beispiele 7) bis 13)**

Einfluß der Katalysatorzusammensetzung

Die Produktqualität bezieht sich auf den Sinter- und den Schmelzpunkt einer Verbindung. Je höher diese Werte liegen, desto reiner ist die Verbindung.

Tabelle 1

| Nr. | 7 (Vergl.) | 8 (Vergl.) | 9 (Vergl.) | 10 | 11 | 12 | 13 (Vergl.) |
|---|---|---|---|---|---|---|---|
| $C_{DX-F6}$ [Mol/kg] | 0,66 | 0,66 | 0,66 | 0,66 | 0,66 | 0,66 | 0,66 |
| $C_{Co}$ [Mol/kg] | - | 0,0150 | 0,0600 | 0,0300 | 0,0150 | 0,0150 | - |
| $C_{Mn}$ [Mol/kg] | 0,0600 | - | - | 0,0300 | 0,0150 | - | 0,0150 |
| $C_{Ce}$ [Mol/kg] | - | - | - | - | 0,0150 | 0,0150 | 0,0150 |
| $\Sigma C_{Metall}$ [Mol/kg] | 0,0600 | 0,0150 | 0,0600 | 0,0600 | 0,0450 | 0,0300 | 0,0300 |
| $C_{Br}$ [Mol/kg] | 0,0150 | 0,0075 | 0,0150 | 0,0150 | 0,0075 | 0,0075 | 0,0075 |
| Rohausbeute [% d.Th.] | 26,5 | 30,8 | 80,5 | 96,2 | 96,2 | 95,0 | <15 |
| Fp. [°C] | 233-235 | 231-234 | 224-236 | 241-242 | 242-244 | 238-241 | - |
| Sintern [°C] | 231 | 218 | 203 | 236 | 237 | 229 | - |
| Reinheit [%] | 82 | 85 | 83 | 95,2 | 98,5 | 93,7 | - |

**Beispiele 14) bis 20)**

Abhängigkeit der Ausbeute und der Produktqualität von der Gesamtionen-Konzentration und vom Verhältnis [Co]:[Mn].

Tabelle 2

| Nr. | 14 (Vergl.) | 15 (Vergl.) | 16 | 17 | 18 | 19 | 20 |
|---|---|---|---|---|---|---|---|
| $C_{DX-F6}$ [Mol/kg] | 0,66 | 0,66 | 0,66 | 0,66 | 0,66 | 0,66 | 0,66 |
| $C_{Co}$ [Mol/kg] | 0,0050 | 0,0050 | 0,0075 | 0,0150 | 0,0225 | 0,0150 | 0,0075 |
| $C_{Mn}$ [Mol/kg] | 0,0050 | 0,0050 | 0,0075 | 0,0075 | 0,0075 | 0,0150 | 0,0225 |
| $\Sigma C_{Metall}$ [Mol/kg] | 0,0100 | 0,0100 | 0,0150 | 0,0225 | 0,0300 | 0,0300 | 0,0300 |
| $C_{Br}$ [Mol/kg] | 0,0025 | 0,0100 | 0,0038 | 0,0056 | 0,0075 | 0,0075 | 0,0075 |
| $C_{Co}$:$C_{Mn}$ | 1:1 | 1:1 | 1:1 | 2:1 | 3:1 | 1:1 | 1:3 |
| Rohausbeute [% d.Th.] | 42,8 | 77,4 | 95,0 | 95,7 | 64,4 | 94,9 | 95,1 |
| Fp. [°C] | 237-239 | 235-237 | 242-243 | 243-245 | 235-238 | 242-243 | 236-240 |
| Sintern [°C] | 234 | 233 | 238 | 237 | 222 | 237 | 222 |

**Beispiele 21) bis 25)**

Einfluß der Bromidkonzentration auf Ausbeute und Produktqualität

Tabelle 3

| Nr. | 21 (Vergl.) | 22 | 23 | 24 | 25 (Vergl.) |
|---|---|---|---|---|---|
| $C_{DX-F6}$ [Mol/kg] | 0,68 | 0,66 | 0,68 | 0,68 | 0,68 |
| $C_{Co}$ [Mol/kg] | 0,0300 | 0,0150 | 0,0280 | 0,0300 | 0,0300 |
| $C_{Mn}$ [Mol/kg] | 0,0300 | 0,0150 | 0,0280 | 0,0300 | 0,0300 |
| $\Sigma C_{Metall}$ [Mol/kg] | 0,0600 | 0,0300 | 0,0560 | 0,0600 | 0,0600 |
| $C_{Br}$ [Mol/kg] | 0,0600 | 0,0075 | 0,0080 | 0,0038 | 0,0015 |
| $C_{DX-F6}/C_{Br}$ | 11,3 | 88 | 85 | 179 | 453 |
| $\Sigma C_M/C_{Br}$ | 1:1 | 4:1 | 7:1 | 16:1 | 40:1 |
| Rohausbeute [% d.Th.] | 94,7 | 94,9 | 92,7 | 94,2 | 71,2 |
| Fp. [°C] | 239,5-241 | 242-243 | 242-244 | 241-243 | 236-240 |
| Sintern [°C] | 235 | 237 | 238 | 236 | 229 |
| organisch geb. Brom [ppm] | 670 | 215 | 152 | 142 | 121 |
| Reinheit [%] | 95,0 | 95,8 | 98,8 | 96,4 | 93,8 |

**Beispiele 26) bis 31)** Einfluß der Wasserkonzentration in der Essigsäure auf die Ausbeute und Reinheit des Produkts

**Tabelle 4**

| Nr. | | 26 | 27 | 28 | 29 | 30 | 31 (Vergl.) |
|---|---|---|---|---|---|---|---|
| $H_2O$-Anfangskonzentration [%] | | 0,4 in 302,5 g Essigsäure | 0,54 in 298 g Essigsäure | 3,4 in 302,5 g Essigsäure | 4,4 in 303 g Essigsäure | 8,4 in 303 g Essigsäure | 15,4 in 303 g Essigsäure |
| Mit DX-F6 dosierte Menge an Eisessig oder Acetanhydrid | | 150 g Ac | 98,9 g $Ac_2O$ 49,4 g Ac | 132,7 g $Ac_2O$ 25,0 g Ac | 150 g Ac | 175 g Ac | 175 g Ac |
| $H_2O$-Endkonzentration | ber. | 6,2 % | 2,7 % | 3,1 % | 8,7 % | 10,7 % | 14,9 % |
| | gef. | 6,5 % | 3,7 % | 4,4 % | 9,3 % | 10,1 % | 14,0 % |
| Fp. [%] | | 242-243 | 242-244 | 242-244 | 241-243 | 239-242 | 222-236 |
| Sintern [°C] | | 237 | 239 | 234 | 227 | 224 | 193 |
| Reinheit [%] | | 95,8 | 98,4 | 97,2 | 93,6 | 91,9 | 85,7 |
| Rohausbeute [% d.Th.] | | 94,7 | 93,0 | 95,0 | 95,3 | 94,9 | 82,4 |
| Reinausbeute [% d.Th.] | | 90,7 | 91,5 | 92,3 | 89,2 | 87,2 | 70,6 |

Ac = Eisessig

$Ac_2O$ = Acetanhydrid

## Patentansprüche

1. Verfahren zur Herstellung einer Verbindung der Formel

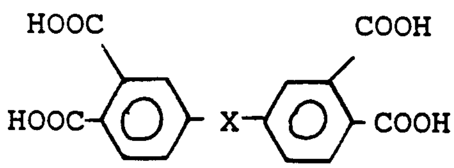

(I)

in der X die Gruppen

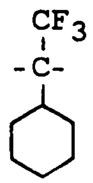
oder
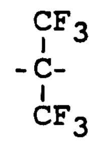

bedeuten
oder deren Anhydrid, durch Luftoxidation in saurem Medium bei erhöhtem Druck und erhöhter Temperatur in Gegenwart eines Katalysatorgemischs, dadurch gekennzeichnet, daß die entsprechende Tetramethylverbindung in einem sauren organischen Medium durch Einleiten von Luftsauerstoff bei Temperaturen von 120 bis 220° C und einem Druck zwischen 5 und 40 bar in Gegenwart von mindestens zwei Schwermetallverbindungen sowie von Bromidionen oxidiert und als solche isoliert wird oder das erhaltene Reaktionsgut in das Dianhydrid der Verbindung der Formel (I) überführt wird.

2. Verfahren nach Anspruch 1, dadurch gekennzeichnet, daß Essigsäure und/oder Propionsäure eingesetzt werden.

3. Verfahren nach Anspruch 1 oder 2, dadurch gekennzeichnet, daß die Reaktionstemperatur 140 bis 190, insbesondere 155 bis 180° C beträgt und daß bei einem Druck zwischen 10 und 30, insbesondere zwischen 14 und 20 bar gearbeitet wird.

4. Verfahren nach einem oder mehreren der Ansprüche 1 bis 3, dadurch gekennzeichnet, daß der zur Oxidation eingesetzte Luftsauerstoff ein Sauerstoffgehalt von über 21 Vol-% besitzt, daß an der Einleitstelle des Sauerstoffs der Sauerstoffpartialdruck mindestens 1 bar, vorzugsweise 2 bis 15, insbesondere 3 bis 10 bar beträgt und daß der Sauestoffpartialdruck in der Gasphase über dem Reaktionsmedium, der sich aus der Formel

$$P_{O_2} = \text{Volumen-\% } O_2 \; (P_{gesamt} - P_{Essigsäuredampfdruck})$$

ergibt, mindestens 0,2 bar, vorzugsweise 0,35 bis 2,8, insbesosndere 0,45 bis 1,3 bar beträgt.

5. Verfahren nach einem oder mehreren der Ansprüche 1 bis 4, dadurch gekennzeichnet, daß als Schwermetallionen Kobalt, Mangan und/oder Cer eingesetzt und diese in Form von Acetatverbindungen zugesetzt werden und daß Brom in Form von Bromiden oder als Lösng von Bromwasserstoff in Wasser oder Eisessig eingesetzt wird.

6. Verfahren nach einem oder mehreren der Ansprüche 1 bis 5, dadurch gekennzeichnet, daß das Mol-Verhältnis von Kobalt zu Mangan gleich 3:1 bis 1:3 ist, wobei die Summe der Konzentrationen der beiden Elemente Kobalt und Mangan gleich 0,01 bis 0,20 g-Atom/kg Gesamtreaktionsmasse, vorzugsweise 0,02 bis 0,12, insbesondere 0,04 bis 0,08 g-Atom/kg beträgt und daß das Mol-Verhältnis der

Summe von Kobalt und Mangan zu Brom 1:0,01 bis 0,8, vorzugsweise 1:0,05 bis 0,4 beträgt.

7. Verfahren nach einem oder mehreren der Ansprüche 1 bis 6, dadurch gekennzeichnet, daß als zusätzliches Metallion Cer im Katalysator enthalten ist im Mol-Verhältnis von der Summe von Kobalt und Mangan zu Cer wie 1:0,02 bis 1,2, vorzugsweise 1:0,05 bis 0,6 und daß das Molverhältnis von Kobalt zu Cer 1:0,02 bis 1,2 beträgt.

8. Verfahren nach einem oder mehreren der Ansprüche 1 bis 7, dadurch gekennzeichnet, daß der Reaktionsansatz nach Beendigung der exothermen Reaktion für 1 bis 3 Stunden auf 150 bis 190° C bei einem Sauerstoffpartialdruck von 0,4 bis 2 bar gehalten wird.

9. Verfahren nach einem oder mehreren der Ansprüche 1 bis 8, dadurch gekennzeichnet, daß die Reaktion bei einer Wasserkonzentration der Monocarbonsäure von 2 bis 12, vorzugsweise 2 bis 7 und insbesondere bei 3 bis 5 % durchgeführt wird.

10. Verfahren nach einem oder mehreren der Ansprüche 1 bis 9, dadurch gekennzeichnet, daß das Dianhydrid unter Verwendung von Acetanhydrid in geringem Überschuß gebildet wird, wobei zur Vervollständigung der Umsetzung Acetanhydrid zugesetzt wird bis zu einem Gehalt von 3 bis 12 % in der Lösung.

11. Verfahren nach einem oder mehreren der Ansprüche 1 bis 10, dadurch gekennzeichnet, daß das Anhydrid gebildet wird:
a) durch destillative Entfernung von Wasser aus der Reaktionslösung bei einer Temperatur oberhalb von 140° C, gegebenenfalls unter Druck, oder
b) durch Erhitzen der Tetracarbonsäure auf 180 bis 190° C unter vermindertem Druck, oder
c) durch destillative Entfernung des Wassers aus der wasserfeuchten Tetracarbonsäure, die in einem mit Wasser nicht mischbaren Lösungsmittel suspendiert ist.

12. Verfahren nach Anspruch 11, dadurch gekennzeichnet, daß als Lösungsmittel Toluol, o-Xylol, Tetralin, Acetophenon oder Diphenylether eingesetzt wird.

13. Verwendung der Verbindungen gemäß Anspruch 1 zur Herstellung von Polykondensaten, insbesondere von Polyimiden, Polycarbonsäureamiden, Polyamidcarbonsäureestern, Polyamiden und Imidoligomeren.

## Claims

1. A process for the preparation of a compound of the formula

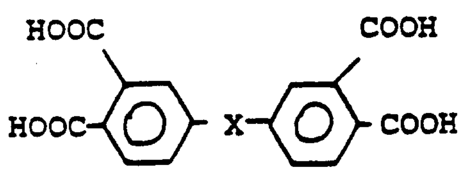

(I)

in which X is the group

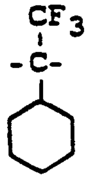

or

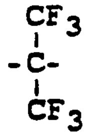

or an anhydride thereof by atmospheric oxidation in an acid medium under increased pressure and at elevated temperature in the presence of a catalyst mixture, which comprises oxidizing the corresponding tetramethyl compound in an acid organic medium by passing in atmospheric oxygen at temperatures of 120 to 220 °C under a pressure of between 5 and 40 bar in the presence of at least two heavy metal compounds and of bromide ions, and isolating the product as such or converting the resulting reaction product into the dianhydride of the compound of the formula (I).

**2.** The process as claimed in claim 1, wherein acetic acid and/or propionic acid is employed.

**3.** The process as claimed in claim 1 or 2, wherein the reaction temperature is 140 to 190 °C, in particular 155 to 180 °C, and the reaction is carried out under a pressure of between 10 and 30, in particular between 14 and 20 bar.

**4.** The process as claimed in one or more of claims 1 to 3, wherein the atmospheric oxygen employed for the oxidation has an oxygen content of more than 21 % by volume, the oxygen partial pressure at the point of entry of the oxygen is at least 1 bar, preferably 2 to 15, in particular 3 to 10 bar, and the oxygen partial pressure in the gas phase above the reaction medium, which results from the equation $P_{O2}$ = % by volume of $O_2$ ($P_{total}$-$P_{acetic\ acid\ vapor\ pressure}$), is at least 0.2 bar, preferably 0.35 to 2.8, in particular 0.45 to 1.3 bar.

**5.** The process as claimed in one or more of claims 1 to 4, wherein cobalt, manganese and/or cerium is employed as heavy metal ions and these are added in the form of acetate compounds, and the bromine is employed in the form of bromides or as a solution of hydrogen bromide in water or glacial acetic acid.

**6.** The process as claimed in one or more of claims 1 to 5, wherein the molar ratio of cobalt to manganese is 3:1 to 1:3, the sum of the concentrations of the two elements cobalt and manganese being 0.01 to 0.20 g atom/kg of total reaction mass, preferably 0.02 to 0.12, in particular 0.04 to 0.08 g atom/kg, and the molar ratio of the sum of cobalt and manganese to bromine is 1:0.01 to 0.8, preferably 1:0.05 to 0.4.

**7.** The process as claimed in one or more of claims 1 to 6, wherein the catalyst contains, as an additional metal ion, cerium in a molar ratio of the sum of cobalt and manganese to cerium of 1:0.02 to 1.2, preferably 1:0.05 to 0.6, and the molar ratio of cobalt to cerium is 1:0.02 to 1.2.

**8.** The process as claimed in one or more of claims 1 to 7, wherein the reaction mixture is kept at 150 to 190 °C under an oxygen partial pressure of 0.4 to 2 bar for 1 to 3 hours after the exothermic reaction has ended.

**9.** The process as claimed in one or more of claims 1 to 8, wherein the reaction is carried out at a water concentration of the monocarboxylic acid of 2 to 12, preferably 2 to 7, and in particular at 3 to 5 %.

**10.** The process as claimed in one or more of claims 1 to 9, wherein the dianhydride is formed using acetic anhydride in a small excess, acetic anhydride being added up to a content of 3 to 12 % in the solution in order to bring the reaction to completion.

**11.** The process as claimed in one or more of claims 1 to 10, wherein the anhydride is formed:
a) by distillative removal of water from the reaction solution at a temperature above 140 °C, if appropriate under pressure, or
b) by heating the tetracarboxylic acid to 180 to 190 °C under reduced pressure, or
c) by distillative removal of the water from the water-moist tetracarboxylic acid, which is suspended in a water-immiscible solvent.

**12.** The process as claimed in claim 11, wherein toluene, o-xylene, tetralin, acetophenone or diphenylether is employed as the solvent.

**13.** The use of a compound as claimed in claim 1 for the preparation of a polycondensate, in particular a polyimide, polycarboxylic acid amide, polyamidocarboxylic acid ester, polyamide or imide oligomer.

## Revendications

1. Procédé pour la préparation d'un composé de formule

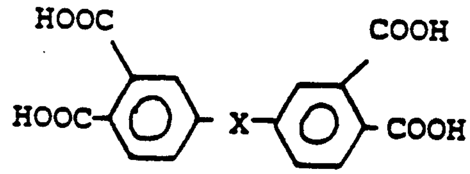

(I)

dans laquelle X signifie les groupes

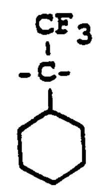

ou

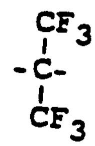

ou son anhydride, par oxydation par l'air en milieu acide, sous pression élevée et à température élevée, en présence d'un mélange de catalyseurs, caractérisé en ce qu'on oxyde le composé tétraméthylique correspondant dans un milieu organique acide, par introduction de l'oxygène atmosphérique, à des températures de 120 à 220 °C et sous une pression de 5 à 40 bars, en présence d'au moins deux composés de métaux lourds ainsi que d'ions de bromure et on l'isole tel quel ou on transforme le produit de réaction en le dianhydride du composé de formule (I).

2. Procédé selon la revendication 1, caractérisé en ce qu'on utilise l'acide acétique et/ou l'acide propionique.

3. Procédé selon les revendications 1 ou 2, caractérisé en ce que la température de réaction est de 140 a 190 °C, notamment de 155 à 180 °C et on travaille à une pression comprise entre 10 et 30, notamment entre 14 et 20 bars.

4. Procédé selon une ou plusieurs des revendications 1 à 3, caractérisé en ce que l'oxygène atmosphérique utilisé pour l'oxydation a une teneur en oxygène supérieure a 21 % en volume, en ce que la pression partielle d'oxygène a l'endroit d'introduction de l'oxygène a l'endroit d'introduction de l'oxygène est d'au moins 1 bar, de préférence de 2 a 15, plus particulièrement de 3 a 10 bars et en ce que la pression partielle d'oxygène dans la phase gazeuse au-dessus du milieu réactionnel, qui résulte de la formule $P_{O2}$ = % en volume $O_2$ ($P_{total}$-$P_{pression\ de\ vapeur\ d'acide\ acétique}$) est d'au moins de 0,2 bar, de préférence de 0,35 à 2,8 bars, de manière particulièrement préférée de 0,45 à 1,3 bar.

5. Procédé selon une ou plusieurs des revendications 1 à 4, caractérisé en ce qu'on utilise en tant qu'ions de métaux lourds le cobalt, manganèse et/ou cérium et on ajoute ceux-ci sous forme de composés de type acétate et on utilise le brome sous forme de bromures ou en tant que solution de bromure d'hydrogène dans l'eau ou acide acétique glacial.

6. Procédé selon une ou plusieurs des revendications 1 à 5, caractérisé en ce que le rapport molaire entre le cobalt et le manganèse est de 3 : 1 à 1 : 3, la somme des concentrations des deux éléments, le cobalt et le manganèse, étant égale à 0,01 à 0,20 g-atome/kg de la masse réactionnelle totale, de préférence de 0,02 à 0,12, notamment de 0,04 à 0,08 g-atome/kg et que le rapport molaire de la somme du cobalt et du manganèse au brome est de 1 : 0,01 à 0,8, de préférence de 1 : 0,05 à 0,4.

7. Procédé selon une ou plusieurs des revendications 1 à 6, caractérisé en ce que le catalyseur contient du cérium en tant que l'ion métallique supplémentaire dans un rapport molaire de la somme du cobalte et du managnèse au cérium de 1 : 0,02 à 1,2, de préférence de 1 : 0,05 à 0,6 et que le rapport molaire

entre le cobalt et le cérium est de 1 : 0,02 à 1,2.

**8.** Procédé selon une ou plusieurs des revendications 1 à 7, caractérisé en ce qu'on maintient la charge réactionnelle après achèvement de la réaction exothermique pendant 1 à 3 heures à une température de 150 à 190 °C pour une pression partielle d'oxygène de 0,4 à 2 bars.

**9.** Procédé selon une ou plusieurs des revendications 1 à 8, caractérisé en ce qu'on met en oeuvre la réaction à une concentration en eau dans l'acide monocarboxylique de 2 à 12, de préférence de 2 à 7 et plus particulièrement de 3 à 5%.

**10.** Procédé selon une ou plusieurs des revendications 1 à 9, caractérisée en ce que le qianhydride se forme en utilisant l'anhydride acétique en faible excès, en ajoutant l'anhydride acétique pour compléter la réaction, jusqu'à une teneur de 3 à 12% dans la solution.

**11.** Procédé selon une ou plusieurs des revendication 1 à 10, caractérisé en ce que l'anhydride se forme :
a) par élimination par distillation de l'eau de la solution réactionnelle à une température supérieure à 140 °C, éventuellement sous pression, ou
b) par chauffage de l'acide tétracarboxylique à une température de 180 à 190 °C sous pression réduite, ou
c) par élimination par distillation de l'eau de l'acide tétracarboxylique humide d'eau, lequel acide est suspendu dans un solvant non miscible à l'eau.

**12.** Procédé selon la revendication 11, caractérisé en ce qu'on utilise en tant que solvant les toluène, o-xylène, tétraline, acétophénone ou éther diphénylique.

**13.** Utilisation des composés selon la revendication 1 pour la préparation de polycondensats, plus particulièrement, des polyimides, amides d'acides polycarboxyliques, esters d'acides polyamidecarboxyliques, polyamides ou oligomères de l'imidol.